# EUROPEAN PATENT APPLICATION

(11) **EP 4 740 976 A1**
(43) Date of publication of application: **13.05.2026**
(21) Application number: 25200720.8
(22) Date of filing: 08.09.2025
(51) Int. Cl.: A61M 1/30, A61M 1/32, A61M 1/36

(54) **ALTERNATING FLOW INTRAVASCULAR CATHETERS AND RELATED TECHNOLOGIES**

(30) Priority: 08.11.2024 US 202463717987 P
(71) Applicant: Critical Innovations, LLC, Lawndale, CA 90260 (US)
(72) Inventor: DONALDSON, Ross I., Lawndale, CA 90260 (US); BUCHANAN, Oliver, Lawndale, CA 90260 (US); YESAYAN, Zhirair, Lawndale, CA 90260 (US); YOUNG, Tyler, Lawndale, CA 90260 (US); SCAGLIONE, Bernard, Lawndale, CA 90260 (US); INGAMELLS, Grant, Lawndale, CA 90260 (US)
(74) Representative: HGF

(57) **Abstract**

An improved method and device for improved catheters and/or related technologies. The device generally comprises an improved medical technology. The provided device substantially improves upon catheter-related medical device technology.

## Description

### Priority Claim

The present application claims the benefit of U.S. Provisional Patent Application No. 63/717,987 filed November 8, 2024, which is hereby incorporated by reference in its entirety.

### Commonly Owned Applications

U.S. Patent No. 9,616,203, filed August 7, 2013 and entitled Method and Device for Simultaneously Documenting and Treating Tension Pneumothorax and/or Hemothorax; U.S.

Patent No. 10,046,147, filed December 23, 2014 and entitled Percutaneous Channel System and Method; U.S. Patent No. 10,814,119, filed August 27, 2018 and entitled Percutaneous Access Pathway System; U.S. Patent No. 11,207,060, filed March 15, 2019 and entitled Systems and Methods Relating to Medical Applications of Synthetic Polymer Formulations; U.S. Patent No. 11,832,833, filed October 5, 2020 and entitled Percutaneous Access Pathway System; U.S. Patent Application Serial No. 17/876,187, filed July 28, 2022 and entitled Wound Treatment Device; U.S. Patent Application Serial No. 18/448,455, filed August 11, 2023 and entitled Percutaneous Access Pathway System; U.S. Patent Application Serial No. 18/485,800, filed October 12, 2023 and entitled Systems and Methods Relating to Medical Applications of Inverse Thermosensitive Polymer Foam Formulations; and U.S. Patent Application Serial No. 18/926,615, filed October 25, 2024 and entitled Autoinjector Assembly are hereby incorporated by reference herein in their entireties.

### US Government License Rights

This invention was made with US Government support under contract N6600124C0031, "Revite^{™} Automated Trauma Resuscitation System," issued by NAVAL INFORMATION WARFARE CENTER PACIFIC. The US Government has certain rights in the invention.

### Technical Field

The present disclosure relates generally to alternating flow technologies, including improved catheter-related medical devices and/or related technologies.

### Background

Extracorporeal Life Support (ECLS) (e.g., extracorporeal membrane oxygenation [ECMO], cardiopulmonary bypass [CPB], extracorporeal cardiopulmonary resuscitation [ECPR]) have advanced dramatically over the last decade and improved the management of patients with refractory respiratory and/or cardiac failure in a variety of settings. Although initial complex and bulky systems were previously limited to specialized quaternary centers, incremental technological improvements have resulted in miniaturized ECLS circuits that allow wider usage, including for a wider range of in-hospital settings (e.g., intensive care unit, emergency department, in-theater military settings) and indications (e.g., extracorporeal cardiopulmonary resuscitation [eCPR], trauma, drug overdose, bridges to therapy). There has been a similar progression of dialysis technologies.

While the main machinery has greatly progressed, deployment of ECLS has continued to be hindered by the need to establish appropriate vascular access with more limited expertise or setting limitations. The use of venovenous (VV) ECLS through a single dual-lumen cannula has simplified some access requirements by requiring only one catheter to be placed. However, this still requires insertion of a very large cannula, as well as in most cases the need for advanced imaging (e.g., fluoroscopy and transesophageal echocardiography [TEE]) to guide and confirm correct cannula position. Unfortunately, this prevents ECLS from being more widely initiated.

In contrast, establishing standard central-line venous access and dialysis catheter access, which utilize smaller catheters than ECLS, is an essential and established skill for a wide range of advanced providers that care for the critically ill (e.g., critical care, emergency, trauma specialists). These procedures are used frequently during critical care resuscitation to establish a route for rapid administration of fluids, blood products, medications, and emergent dialysis. These procedures can also be performed in conjunction with point-of-care (POC) ultrasound, which is now widely available and has been demonstrated to improve procedural success and reduce complications. POC ultrasound has a very small logistical footprint, with some handheld versions currently weighing ~140g (0.3lbs) and medics have been shown to be capable of using this technology in the out-of-hospital setting. This distinguishes POC ultrasound from TEE and fluoroscopy, which require specialized skills to deploy and have very large and unwieldy equipment.

As aforementioned, the prior art demonstrates that VV-ECLS is of benefit and can be performed through a single dual-lumen cannula (typically 23-31 Fr) in the internal jugular vein (IJV) (e.g., Avalon Elite Catheter by Getinge). This simplifies the ECLS procedure by requiring cannulation of only one vessel. However, blood flow in this circuit is directly determined by the limited, fixed drainage capacity of its catheter (most frequently its venous intake), thus requiring a very large catheter size, with subsequent increased complication risk and procedural difficulty of placement.

For this setup, a single double-lumen cannula is inserted into the right IJV, passing through the superior vena cava (SVC) and right atrium (RA) and into the inferior vena cava (IVC). Proximal and distal holes connect to its intake lumen and simultaneously remove deoxygenated blood from the upper and lower body. Oxygenated blood from the ECLS circuit is returned through an exit port that is directed at the tricuspid valve (TV). This delivery port should be positioned in the RA and rotated ideally to direct flow across the TV to minimize recirculation. Recirculation phenomenon occurs when effluent blood is re-aspirated back into the ECLS circuit, greatly reducing the efficiency of VV-ECLS. As aforementioned, appropriate positioning of the cannula is currently performed with advanced imaging to confirm the position of the wire at each step of the procedure, as well as the positioning of the catheter at completion.

The prior art also includes portable ECLS systems. One example is the Breethe OXY-1 System^{™} (Abiomed^{®}). This system has received FDA 510(k) clearance for ECMO up to 6 hours. It consists of two modules: a pump lung unit (PLU) and a respiratory management unit (RMU). The PLU contains an integrated centrifugal pump and hollow fiber membrane oxygenator consisting of 30,000 polymethylpentene fibers, each with a diameter of 380 microns. The fibers are woven into a cylindrical structure surrounding the centrifugal pump, which acts to draw blood from the patient through the fiber mesh and return the now oxygenated blood back to the patient. The RMU can deliver O₂ via dedicated tanks (External Gas Mode) or directly using the in-built oxygen concentrator (Internal Gas Mode). Additionally, the RMU houses a power supply and control interface. This demonstrates that ECLS systems can be miniaturized for portability, although currently available hardware has a limited duration of use (≤6 hours) due to the risk of thrombotic complications.

Prior attempts at venous drainage from the IVC have resulted in reduced performance due to collapsing of the IVC wall onto the catheter, which inhibits drainage and results in suction events (i.e., "chattering").

The literature discloses various additional known methods and devices related generally to the field of medicine and more particularly to improved catheters and/or related technologies. However, all are limited in some aspect.

### Summary

The present disclosure overcomes and substantially alleviates the deficiencies in the prior art by providing improved devices and methods related generally to the field of medicine and more particularly to alternating flow technologies, including improved catheters, ECLS systems, dialysis systems, and/or related technologies. Under various embodiments, the present disclosure is directed to a catheter-related medical device. Under embodiments, the catheter-related medical device is an improved system, machine, vascular catheter (e.g., intravenous catheter, peripheral catheter, central venous catheter, tunneled catheter, implanted port catheter, arterial catheter, venous catheter, dialysis catheter, ECMO catheter, ECLS catheter, CPB machine, and/or peripherally inserted central catheter), and/or related medical equipment. In embodiments, the improved vascular catheter and/or catheter-related medical device allows for improved flow rates and/or alternating one-way flow (i.e., flow in one direction followed by flow in the other direction at least partially in the same lumen of a catheter) in comparison to the prior art. In embodiments, the device provides an improvement in drawing fluid through it (e.g., drawing blood for laboratory analysis from a peripheral vein; blood withdrawal/drainage during ECLS or dialysis).

In embodiments, a catheter-related medical device disclosed herein is designed to allow alternating phases of drainage and return flow (e.g., bidirectional) through a single catheter lumen. In embodiments, this provides the benefit of being able to utilize the maximum and/or near maximum functional cross-sectional area of the lumen to facilitate maximal flow (e.g., bidirectional flow through a single catheter lumen). In embodiments, this design increases catheter flow (e.g., up to 200%) in comparison to a traditional dual-lumen catheter through the important insight that flow through a single lumen is dramatically greater than the flow through that same area when divided into more than one lumen (e.g., as occurs in traditional continuous flow dual-lumen catheters, such as for single-catheter ECLS and dialysis systems that traditionally have continuous and not alternating flow). As demonstrated by the Hagen-Poiseuille Equation, flow through a given cross-sectional area can be much greater than the sum of its parts, as the volumetric flow rate (Q) is directly proportional to the cross-sectional area of the catheter squared (A²).

In embodiments, a catheter-related medical device disclosed herein provides alternating flow to a patient through a single catheter and/or single catheter lumen (e.g., a cycle of flow in one direction followed by a cycle of flow in the other direction). In embodiments, the catheter-related medical device provides ECLS to a patient and the alternating flow is a cycle between oxygenated and deoxygenated blood through a single catheter (e.g., placed in the femoral vein) and/or single catheter lumen. This is novel because all existing ECLS machines work through a continuous flow of drainage and return to a patient either through a single dual-lumen catheter or at least two single lumen catheters, thus teaching away from the current invention. In embodiments, the catheter-related medical device provides dialysis to a patient and the alternating flow is a cycle between withdrawn and returned blood through a single catheter and/or single catheter lumen.

In embodiments, the catheter-related medical device contains components utilized in traditional ECLS and/or dialysis systems modified to allow alternating (instead of continuous) flow to the patient, for example one or more of the following: catheter(s), tubing(s), user interface(s), gas blender(s), pump(s), bubble trap(s), oxygenator(s), heat-exchanger(s), perfusion circuit(s), filter(s), actuator(s), reservoir(s), blood analyzer(s), alarm(s), battery(ies), processor(s), control algorithm(s), heparin pump(s), sensor(s), dialyzer(s), dialysate delivery system(s), heparin pump(s), and/or ultrafiltration control system(s).

In embodiments, the catheter-related medical device includes one or more reservoirs to allow the storage of drained blood and/or other medical fluids in the system (e.g., during its drainage phase). At least a portion of this volume is then returned to the patient during its return phase. In embodiments, this includes a hard-shell reservoir. In embodiments, this includes a soft bladder reservoir. In embodiments, the system utilizes one or more sensors to determine volume in the reservoir. In embodiments, these sensors directly or indirectly sense fluid level, pressure, displacement, flow, and/or weight through the use of optical, conduction, ultrasonic, infrared, capacitive, and/or weight-based methods to determine absolute and/or relative volumes and/or indirect inferences thereof.

In embodiments, the catheter-related medical device includes one or more pumps to drive fluids through the system. In some embodiments, this is a single centrifugal pump, which minimizes potential mismatch between pulled and pushed fluids in the system. In some embodiments, this is two or more centrifugal pumps, to maintain flow in more than one perfusion circuit. Various embodiments include other various pumps, examples of which include: centrifugal, diaphragm, piston, submersible, gear, positive displacement, peristaltic, screw, lobe, axial-flow, dynamic, horizontal centrifugal, reciprocating, vertical centrifugal, displacement, progressive cavity, vane, gear, reciprocating, self-priming, horizontal multistage, and/or rotary.

In embodiments, the catheter-related medical device includes one or more perfusion circuits. In embodiments, the perfusion circuit is disposable. In embodiments, the perfusion circuit consists functionally of two connected circuit loops: one withdrawing blood and/or other medical fluids from the body and storing them within the reservoir (i.e., drainage loop) and one withdrawing blood and/or other medical fluids from the reservoir and infusing them into the body (i.e., return loop). In embodiments, the perfusion circuit consists functionally of two connected circuit loops similar to human physiology: one withdrawing blood and/or other medical fluids from the body and later returning them to the body (i.e., systemic loop) and one withdrawing blood and/or other medical fluids from the systemic loop and sending it to the oxygenator and/or dialyzer (i.e., pulmonary and/or renal loop). In embodiments, the catheter-related medical device functions as a closed-loop system, thus the amount of fluid drained or returned to the patient is roughly equivalent to the amount drained or returned to the reservoir. In embodiments, the catheter-related medical device is additionally capable of functioning as an open-loop system, where blood and/or other medical fluids and/or medications may be added to or withdrawn from the system (e.g., giving additional blood product and/or other medical fluids to the patient through the system). In embodiments, part or all of the perfusion circuit and/or other catheter-related medical device components are coated and/or embedded with one or more beneficial substances, examples of which include: heparin-bonded (e.g., CBAS/Carmeda; MAQUET Bioline), phosphorylcholinebased "biomimetic" coatings (e.g., Phisio), Albumin-heparin hybrids (e.g., Bioline), NO-releasing and/or NO-catalyzing coatings, zwitterionic and/or hydrogel antifouling surfaces (e.g., PEG-like, SBMA), omniphobic and/or tethered-liquid perfluorocarbon surfaces, and/or antimicrobialimpregnated (e.g., chlorhexidine-silver sulfadiazine).

In embodiments, sensor(s) include those sensing for one or more of the following: flow, pressure, air and/or bubbles, oxygen saturation, oxygen, carbon dioxide, gas exchange, temperature, conductivity, hematocrit and/or hemoglobin, viscosity, clot, electrolytes, biochemical, optical and/or photometric, fluid level, and/or others. In embodiments, sensors are placed on one or more circuit loops to determine relations between loops in the system.

In embodiments, the user interface(s) and/or control algorithm(s) allow control of the system by setting target reservoir volume, pump motor speed, and/or the relative closure and/or resistance of one or more loop actuators. In embodiments, system control includes applying different settings for loops actuator(s) during different system phases (e.g., withdrawal, return, setup, idle). In embodiments, one or more loop actuators are capable of varying the relative resistance between tubing sections, so as to preferentially direct flow into desired circuit loops depending on system phase. In embodiments, this includes full closure of one or more tubing sections by one or more loop actuators. In embodiments, loop actuator(s) do not fully close and instead maintain at least a minimum flow through circuit loops to prevent and/or minimize clotting, hemolysis, water hammer, and/or other deleterious effects to blood and/or other medical fluids. In embodiments, loop actuator(s) are any actuators and/or valve mechanisms known in the art that can regulate the flow of fluid through a tube or related structure. In various embodiments, the loop actuator(s) are: pinch, solenoid, diaphragm, needle, globe, ball, rotary, gate, proportional, and/or check valve(s) and/or specialized microfluidic actuators (e.g., piezoelectric, electroosmotic, shapememory alloy).

In embodiments, a catheter-related medical device disclosed herein is an alternating-flow catheter, a control system, and/or inputs and/or consumables alone or in combination. In embodiments, the catheter-related medical device provides gas-exchange, dialysis, and/or trauma resuscitation through a single reduced-size (e.g., <15 Fr) intravascular cannula. In embodiments, use of an alternating-flow catheter in comparison to a traditional (e.g., dual-lumen) catheter results in increased catheter flow. In embodiments, the catheter-related medical device results in decreased drainage insufficiency, decreased recirculation, and/or simplified insertion. In embodiments, alternating-flow does not mean that the fluid flows fully through the same channel or lumen within the device, but rather that the catheter is utilizing the near-maximal inner functional cross-sectional area of the outer catheter for both phases: drainage (i.e., fluid leaving the body through the catheter) and return (i.e., fluid entering the body through the catheter).

In embodiments, an alternating-flow catheter disclosed herein has an outer catheter with one or more distal axial hole(s) used for delivering return fluid and one or more proximal holes for fluid drainage. In embodiments, the inner diameter of the outer catheter is the maximal inner functional cross-sectional area of the catheter. In embodiments, the alternating-flow catheter achieves near-maximal inner functional cross-sectional area usage for both drainage and return by inclusion of a collapsible inner wall and/or sheath that prevents the drained fluid (e.g., blood) from mixing with the return fluid (e.g., blood, therapeutic) when within the catheter. In embodiments, when combined with one or more check valves, this minimizes and/or prevents the mixture of oxygenated blood with deoxygenated blood when used for ECLS (i.e., recirculation).

In embodiments, an alternating-flow catheter disclosed herein achieves near-maximal inner functional cross-sectional area usage for both drainage and return by use of an inner catheter caused to move within an outer catheter during the return phase, so as to cover the proximal drainage holes for fluid. During the return phase, the inner catheter moves so as to unseal the proximal drainage holes to allow drainage. In embodiments, this inner catheter movement is caused at least partially by the movement of fluid within the system. In embodiments, this inner catheter movement is caused at least partially through direct actuation (e.g., an electronic motor causing the movement).

In embodiments, an alternating-flow catheter disclosed herein achieves near-maximal inner functional cross-sectional area usage for both drainage and return by use of an inner catheter that moves proximally and distally. In embodiments, an alternating-flow catheter disclosed herein achieves near-maximal inner functional cross-sectional area usage for both drainage and return by use of an inner catheter that is a collapsable tube that has a reduced cross-sectional area when stretched and a larger cross-sectional area when relaxed.

In embodiments, an alternating-flow catheter disclosed herein achieves near-maximal inner functional cross-sectional area usage for both drainage and return by use of an inner catheter with holes that can match to an outer catheter's proximal drainage holes when in a particular configuration. A phase-dependent twisting motion on the inner catheter can cause the occlusion of the outer proximal drainage holes during the return phase and the unsealing of these holes during the drainage phase.

In embodiments, an alternating-flow catheter disclosed herein achieves near-maximal inner functional cross-sectional area usage for both drainage and return by use of an inner catheter with holes that can match to an outer catheter's proximal drainage holes when in a particular configuration. In this embodiment, a phase-dependent proximal-distal motion on the inner catheter can cause the occlusion of the outer proximal drainage holes during the return phase and the unsealing of these holes during the drainage phase.

In embodiments, an alternating-flow catheter disclosed herein has one or more balloons outside the catheter that inhibit vascular collapse (e.g., chattering) and/or reduce recirculation. In embodiments, the alternating-flow catheter has one or more distal check valves that reduce recirculation within the device. In embodiments, the alternating-flow catheter has the benefit of minimizing thrombotic risk by preventing areas of stagnated flow through alternating delivery and/or minimizing damage to blood cells by using the full catheter diameter (e.g., reduced shear force from increased flow).

In embodiments, a catheter-related medical device disclosed herein utilizes a VV-femoral setup (e.g., to provide faster, safer, and/or more reliable access). In embodiments, the catheter-related medical device decreases drainage insufficiency, which occurs when there is insufficient venous return or excessively negative drainage pressure. Since blood flow in a VV-ECLS circuit is directly limited by drainage capacity, this is of key importance. In embodiments, the catheter-related medical device is placed via the Seldinger technique. In embodiments, the catheter-related medical device improves venous drainage in part by having one or more intake ports (e.g., spanning a ~20 cm intake length). In embodiments, the catheter-related medical device utilizes fluid pressures ranging from -300 mmHg to 300 mmHg and/or a subset within.

In embodiments, the catheter-related medical device allows the user and/or a control algorithm to change the time duration that the system is in drainage and/or return, which allows tailoring of these phases directly to patient physiology in real-time. In embodiments, a catheter-related medical device's alternating flow setup allows system flexibility when attempting to match venous return to arterial flow, which must be equal in a closed ECLS loop. Traditional multi-lumen or two-catheter systems have fixed cross-sections and constant flow, thus when drainage insufficiency occurs pump speed must be reduced and is limited typically to the fixed venous return. However, in embodiments the catheter-related medical device has the ability to vary the time duration and/or individual pressure gradients of the return and drainage phases, thus allowing more plasticity in titrating settings that maximize system delivery in real time for a given patient. In embodiments, the catheter-related medical device operates in two phases (e.g., drainage of deoxygenated blood and return of oxygenated blood) that occur serially. In embodiments, the catheter-related medical device can independently vary its overall cycle time and/or drainage:return ratio. In embodiments, the catheter-related medical device uses a software algorithm based of internal sensors to optimize flow.

In embodiments, a catheter-related medical device disclosed herein minimizes recirculation by having a distance along the tube from drainage and return holes and/or use of an at least partially occlusive balloon around the outer catheter. In embodiments, the catheter-related medical device's pulsatile flow decreases recirculation and/or clotting, as it is more physiologic and synergistic with human physiology.

In embodiments, a catheter-related medical device disclosed herein unifies intravascular access for a patient through a single intravascular device (e.g., fluid resuscitation, medication administration, blood sampling). Normally, multi-lumen tubes are required for therapeutic delivery to prevent adverse mixing within the catheter. For example, both calcium and blood products are indicated in trauma, but cannot be infused together. Different intravenous (IV) lines add to provider workload, while multi-lumen catheters exhibit the aforementioned substantial decreases in flow. However, in embodiments the catheter-related medical device functions in a pulsatile manner, functionally clearing catheter of a particular product(s) more swiftly.

In embodiments, a catheter-related medical device disclosed herein utilizes biocompatible materials, minimizes wall thickness (e.g., by use of coated wire and/or inner braid), uses a lubricious coating to increase flow (e.g., PVP, PTFE, fluoropolymer), and/or uses a coating to prevent clotting in the heparin-free system (e.g., PEO, albumin, pyrolytic carbon, phosphorylcholine, elastin-based). In embodiments, the catheter-related medical device uses a catheter with one or more of the following: non-braided, braided, translucent, straight, curved, tapered, low profile, lament, PEEK, ultra-high weight molecular polyethylene, fiberglass, carbon fiber, PET, platinum, iridium, tungsten, stainless steel, Kevlar^{™}, nitinol, radiopaque, silicon, polyurethane, polyvinyl chloride, polyethylene, polytetrafluorethylene, braid-reinforced, coilreinforced, longitudinal wire reinforcement, thin-walled, lubricious coating, anti-clotting, ultrathin liners, PTFE-lined, hydrophilic coating, single lumen, multi-lumen, multi-durometer, marker band(s), and/or radiopaque filler(s).

In embodiments, a catheter-related medical device disclosed herein includes a control system. In embodiments, the control system is portable, rugged, airworthy, and/or self-contained. In embodiments, the control system is an ECLS and/or dialysis system adapted to provide pulsatile (instead of continuous) flow.

In embodiments, a catheter-related medical device disclosed herein is an ECLS system that has one or more of the following characteristics:
- Facilitates single site VV-ECLS cannula placement with a smaller catheter (e.g., ≤15 Fr).
- Does not require confirmation of cannula placement by any bulky means (e.g., fluoroscopy).
- Is designed for prehospital use with ruggedization and reduced size, weight, and power.
- Makes cannulation easier to perform, thus allowing deployment by nonsubspecialist providers.
- Utilizes closed-loop control algorithms for fluid, medication, and gas-exchange administration, with associated intelligent safety features.
- Provides both trauma resuscitation and/or blood oxygenation.
- Can be used for longer durations (e.g., ≥48 hours) with improved oxygen transfer and minimization of thrombotic risk.

In embodiments, a catheter-related medical device disclosed herein provides: (1) sustained VV-ECLS; (2) rapid resuscitative infusion; (3) ongoing pressor support (e.g., continuous minibolus doses of pressors); and/or (4) automated administration of other beneficial medications (e.g., TXA, calcium). In embodiments, the catheter-related medical device can treat hemorrhagic shock in an automated and/or semi-automated fashion. In embodiments, the catheter-related medical device allows rapid resuscitative infusion (e.g., of warmed whole blood,) through the alternating-flow catheter. In embodiments, delivery is controlled by closed-loop algorithms. In embodiments, the catheter-related medical device contains one or more sensors (e.g., pressure, temperature, laboratory test). In embodiments, the catheter-related medical device automates critical care and provides closed-loop administration of pressors and other therapeutic medications with minimal human oversight.

There have been illustrated and described herein methods and devices related to medical technology. While particular embodiments of the disclosure have been described, it is not intended that the disclosure be limited thereto, as it is intended that the disclosure be as broad in scope as the art will allow and that the specification be read likewise.

While the catheter-related medical device disclosed herein has been described with a certain degree of particularity, it is manifest that many changes may be made in the details of construction and the arrangement of components without departing from the spirit and scope of this disclosure. It is understood that the disclosure is not limited to the embodiments set forth herein for purposes of exemplification and that elements of certain embodiments can be combined with elements of other embodiments. Additional objects, advantages, and novel features of the disclosure will be set forth in the description which follows, and will become apparent to those skilled in the art upon examination of the following detailed description and figures. It should be understood that not all of the features described need be incorporated into a given system or method.

### Brief Description of the Drawings

FIG. 1 is an isometric view of a catheter-related medical device in accordance with an embodiment of the disclosure.
FIG. 2 is a cross-sectional view of a catheter-related medical device in accordance with an embodiment of the disclosure.
FIG. 3 is an isometric view of a catheter-related medical device in accordance with an embodiment of the disclosure.
FIG. 4 is a cross-sectional view of a catheter-related medical device in accordance with an embodiment of the disclosure.
FIG. 5 is an isometric view of a catheter-related medical device in accordance with an embodiment of the disclosure.
FIG. 6 is a schematic view of a catheter-related medical device in accordance with an embodiment of the disclosure.
FIGS. 7-11 are schematic views of catheter-related medical devices in accordance with embodiments of the disclosure.

### Detailed Description

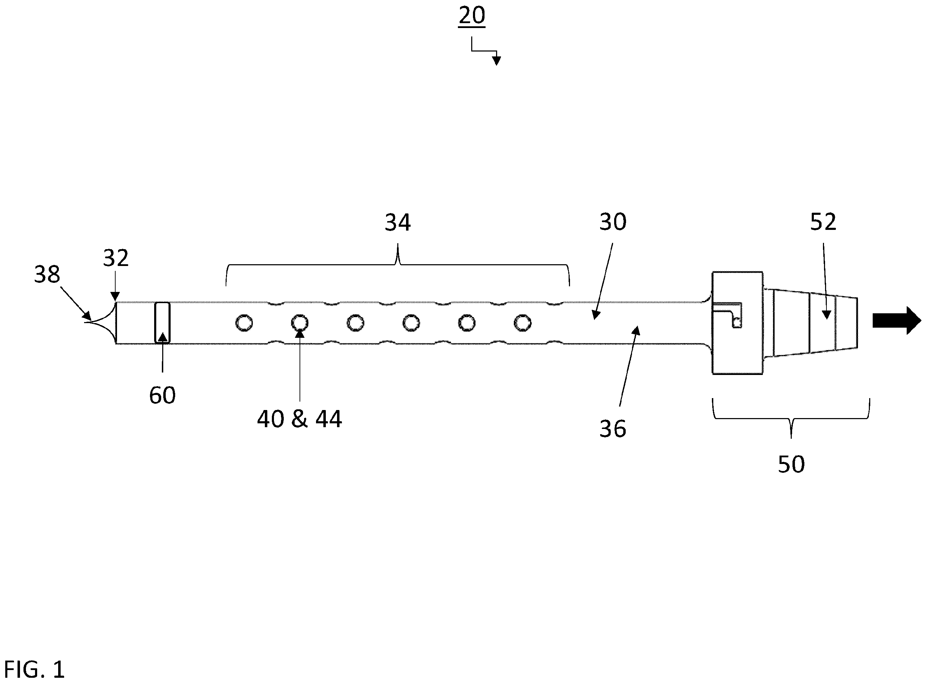

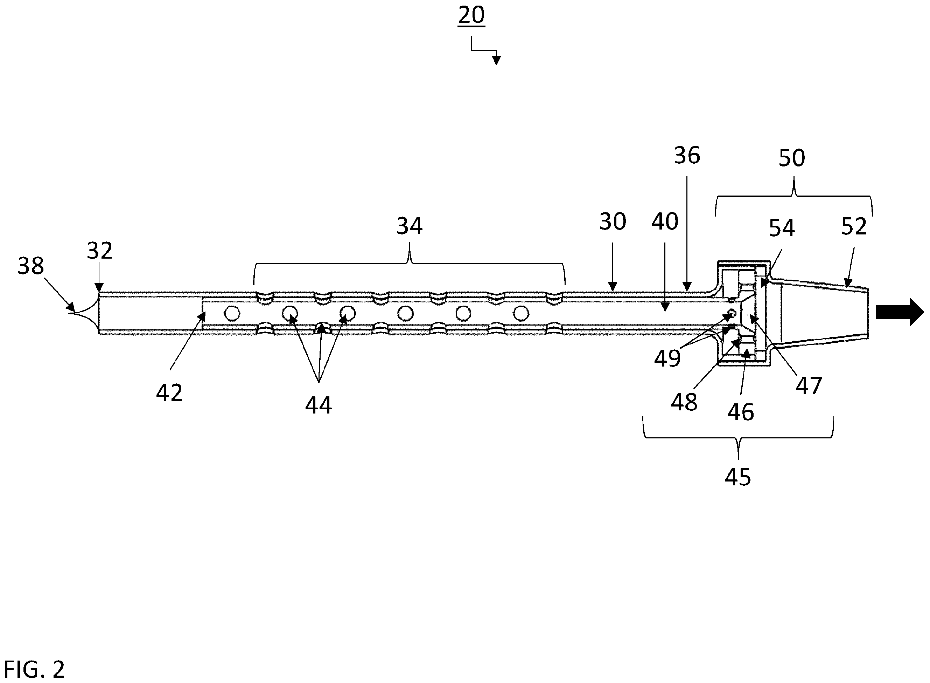

Referring to the drawings, Figs. **1-2** generally illustrate one embodiment of part of the present disclosure. Under this embodiment, an alternating-flow catheter **20** is shown, made up of outer catheter **30,** inner catheter **40,** catheter cap **50,** and one or more balloon(s) **60.** In this embodiment, outer catheter **30** further contains one or more distal axial hole(s) **32,** one or more proximal hole(s) **34,** securing area **36,** and check valve **38.** In this embodiment, inner catheter **40** further contains one or more distal axial hole(s) **42,** one or more proximal hole(s) **44,** and alternating-flow control mechanism **45.** Alternating flow control mechanism **45** further contains alternating flow actuator **46,** one or more main flow channel(s) **47,** one or more accessory flow channel(s) **48,** and one or more catheter return hole(s) **49.** In this embodiment, catheter cap **50** further contains tubing connector **52** and gasket **54.** Some embodiments do not include balloon **60.** In some embodiments, tubing connector **52** is a standard luer connector (e.g., male or female).

In this embodiment, alternating-flow catheter **20** is shown as it is drawing fluid from the body (i.e., drainage phase). Tubing connected from the rest of the system (not shown in these figures) connects to tubing connector **52** and in this phase causes a vacuum to form within the middle of catheter cap **50.** In these figures, this has caused alternating-flow control mechanism **45** to move to its proximal position in reference to catheter cap **50** and outer catheter **30.** Catheter return hole(s) **49** and accessory flow channel(s) **48** in conjunction have ensured that alternating flow actuator **46** can move freely to this proximal position, without movement actuation inhibited by a buildup of a vacuum between the distal side of alternating flow actuator **46** and outer catheter **30** and/or catheter cap **50.** Some embodiments do not include catheter return hole(s) **49** and/or accessory flow channel(s) **48.**

Because alternating-flow control mechanism **45** is in its proximal position, proximal hole(s) **44** on inner catheter **40** are in line with proximal hole(s) **34** on outer catheter **30.** This allows fluid outside the catheter (e.g., blood in a vascular structure) to be drawn into the catheter through these holes. Further, the vacuum within the device causes check valve **38** on outer catheter **30** to be in its closed position, preventing fluid from being drawn in from the distal axial hole(s) **32** (e.g., to reduce recirculation when used for ECLS). Some embodiments do not include check valve **38.**

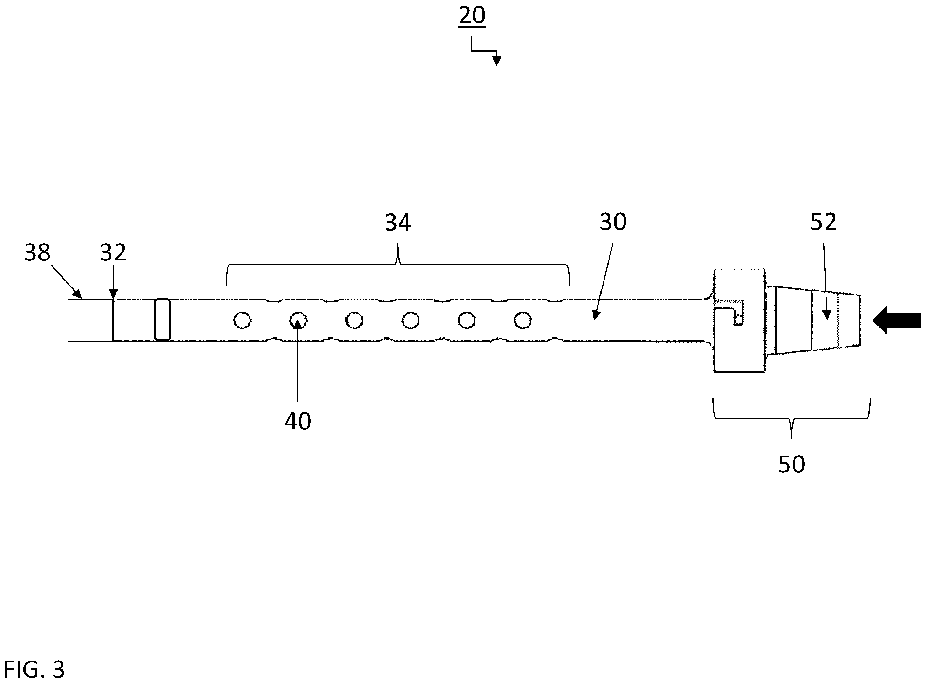

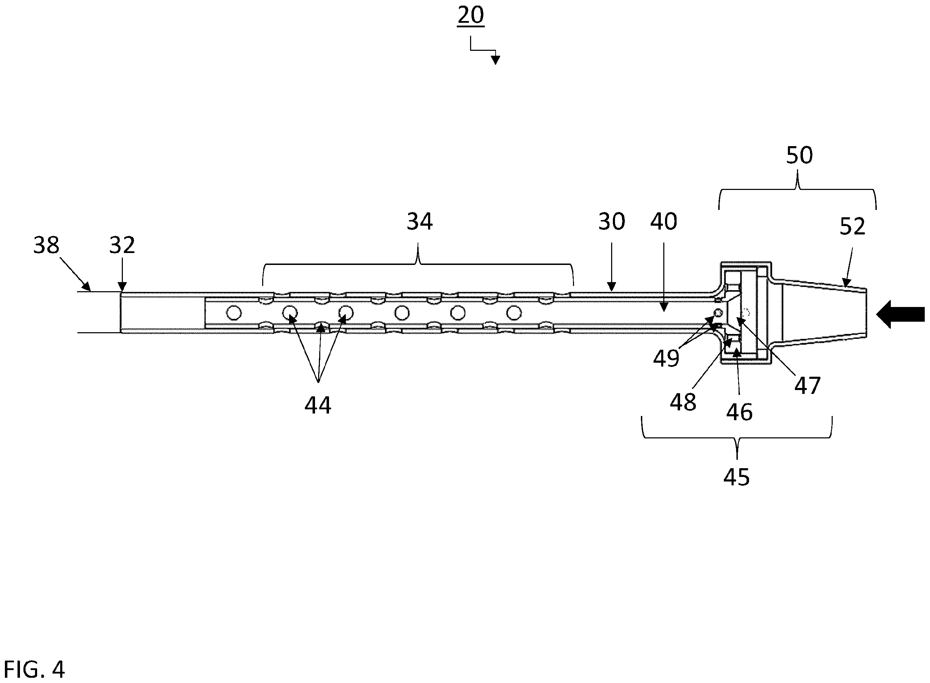

Figs. **3-4** generally illustrate alternating-flow catheter **20** as it is delivering fluid into the body (i.e., return phase). Tubing connected from the rest of the system (not shown in these figures) connects to tubing connector **52** and in this phase causes pressure to build within the middle of catheter cap **50.** In these figures, this has caused alternating-flow control mechanism **45** to move to its distal position in reference to catheter cap **50** and outer catheter **30.** Catheter return hole(s) **49** and accessory flow channel(s) **48** in conjunction have ensured that alternating flow actuator **46** can move freely to this distal position, without movement actuation inhibited by a buildup of pressure between the distal side of alternating flow actuator **46** and outer catheter **30** and/or catheter cap **50.** Some embodiments do not include catheter return hole(s) **49** and/or accessory flow channel(s) **48.**

Because the alternating-flow control mechanism **45** is in its distal position, Proximal hole(s) **44** on inner catheter **40** are not in line with proximal hole(s) **34** on outer catheter **30** (i.e., the inner catheter **40** occludes the proximal hole(s) **34** on outer catheter **30**). This prevents fluid inside the catheter (e.g., blood and/or therapeutic fluid) from flowing through the proximal holes **34** of outer catheter **30.** Instead, the pressure within the device causes check valve **38** on outer catheter **30** to open and fluid to eject only from the distal axial hole(s) **32.** Some embodiments do not include check valve **38.**

It should be apparent to those skilled in the art upon examination of the above figures that alternating-flow catheter **20** could achieve its goal of facilitating proximal withdrawal and distal return of blood and/or other medical fluids utilizing other mechanisms beside the proximal and distal movement of inner catheter **40** in relation to outer catheter **30.** In embodiments, this goal is instead achieved through a rotational motion between inner catheter **40** in relation to outer catheter **30,** via multiple miniature check valves over proximal hole(s) **44** and/or proximal holes **34,** and/or via other related mechanisms. In embodiments, alternating-flow control mechanism **45** is at least partially actuated directly via the flow and/or pressure of blood and/or other medical fluids in the system. In embodiments, alternating-flow control mechanism **45** is partially or fully actuated directly from the control system (e.g., via direct electrical wiring, wireless, remote control, fieldbus and industrial communication protocol(s), hydraulic, pneumatic, optical, acoustic, embedded and smart actuator interfaces and/or controls). In embodiments, such alternating-flow control mechanism actuators include electromagnetic actuators (e.g., solenoids, electromagnetic relays/clutches, voice coil actuators, electric motors), electrostatic actuators (e.g., mems devices, precision positioning plates), piezoelectric actuators (e.g., ultrasonic transducers, precision optical positioners), thermal actuators (e.g., shape memory alloys, bimetallic strips, thermal expansion actuators), hydraulic actuators (e.g., cylinders/pistons, hydraulic motors), pneumatic actuators (e.g., pneumatic cylinders, rotary pneumatic actuators, soft pneumatic actuators), and/or mechanical/energy-storing actuators (e.g., springs, cams, linkages, magnetic shape memory alloys).

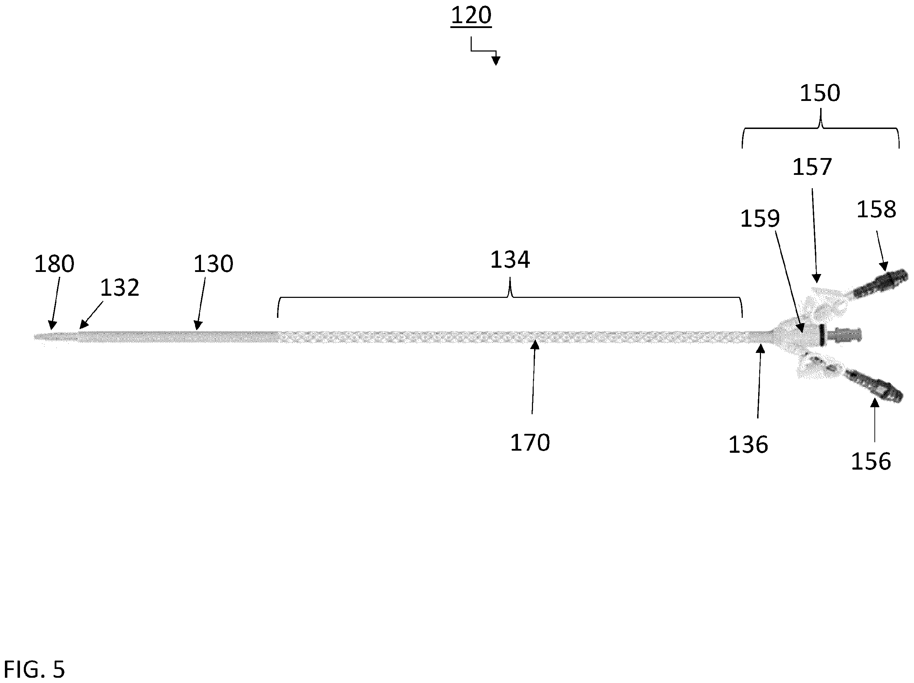

Fig. **5** generally illustrates another embodiment of part of the present disclosure. Under this embodiment, alternating-flow catheter **120** achieves near-maximal inner functional cross-sectional area usage for both drainage and return by inclusion of collapsible inner wall and/or sheath **170.** Under this embodiment, alternating-flow catheter **120** is shown, made up of outer catheter **130,** collapsible inner wall and/or sheath **170,** and catheter cap **150.** In this embodiment, outer catheter **130** further contains one or more distal axial hole(s) **132,** one or more proximal hole(s) **134,** and securing area **136.** In this embodiment, catheter cap **150** further contains drainage port **156,** one or more port clamp(s) **157,** return port **158,** and hub with hemostatic valve **159.** To facilitate placement into the vasculature, this embodiment additionally contains dilator **180** for use with the Seldinger technique. Drainage port **156** allows fluid (e.g., deoxygenated blood) to be removed from alternating-flow catheter **120.** Return port **158** allows fluid (e.g., oxygenated blood) to be put into alternating-flow catheter **120.** One or more port clamp(s) **157** allows the clamping of drainage port **156** and/or return port **158.** Hub with hemostatic valve **159** allows placement of dilator **180** into alternating-flow catheter **120** during setup and/or provides a check valve function when dilator **180** is removed that prevents fluid from exiting alternating-flow catheter **120** (e.g., for use with the Seldinger technique placement).

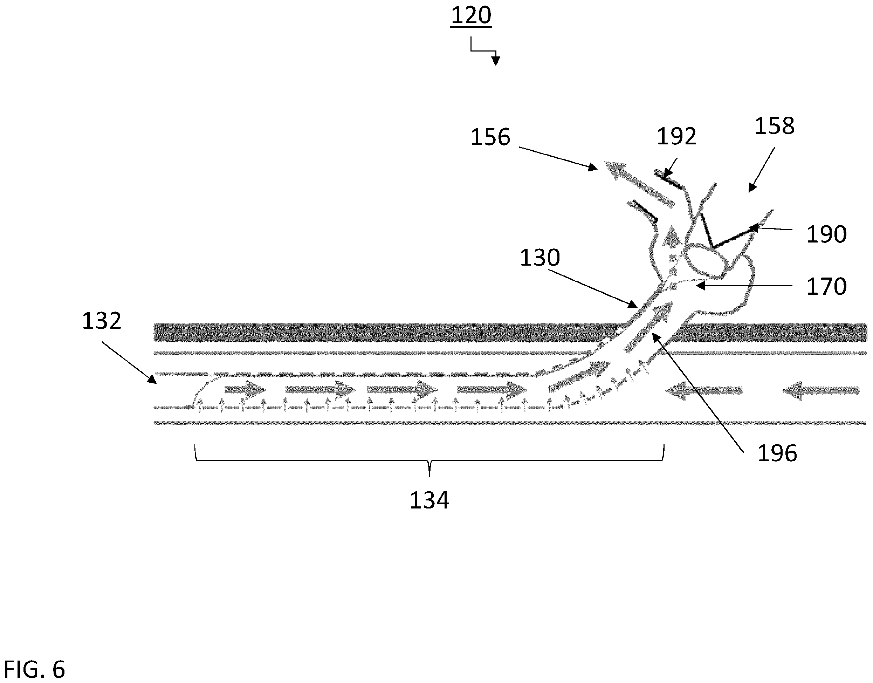

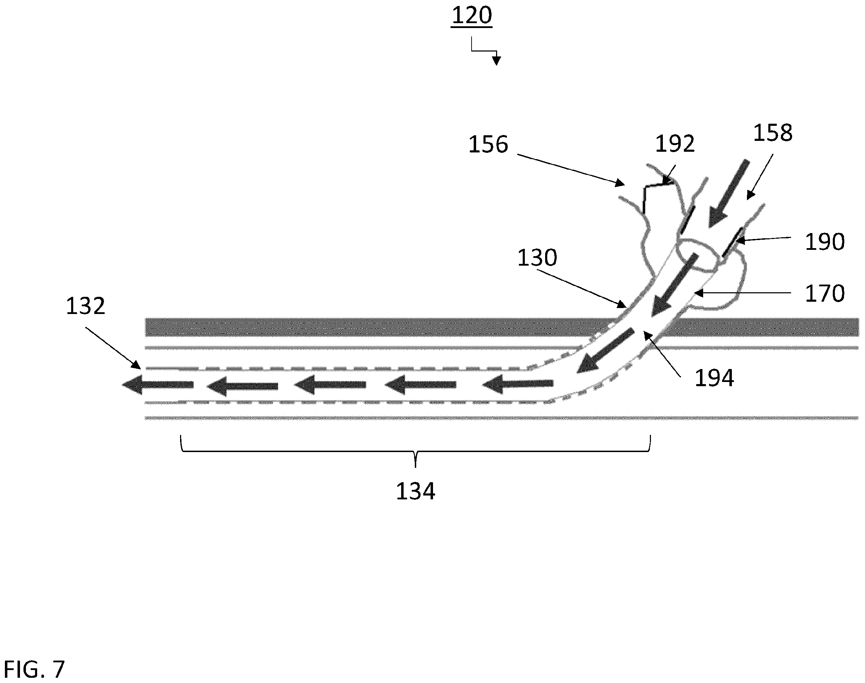

Referring to Figures **6-7****,** alternating-flow control is performed in this embodiment by collapsible inner wall and/or sheath **170** in conjunction with check valves **190** and **192.** When a vacuum is placed on drainage port **156** (Fig. **6**), check valve **192** opens and inner wall and/or sheath **170** collapses. This allows fluid to be drawn through channel **196** made by inner wall and/or sheath **170** collapsing against one side of outer catheter **130** that further exposes one or more proximal hole(s) **134** to allow fluid (e.g., deoxygenated blood) to be drawn into the alternating-flow catheter **120.** When pressurized fluid (e.g., oxygenated blood) is placed into return port **158** (Fig. 7), check valve **190** opens and inner wall and/or sheath **170** expands. This allows fluid to be pushed through channel **194** made by inner wall and/or sheath **170** expanding within outer catheter **130** and, closing off the pathway to the one or more proximal hole(s) **134** and exposing the pathway to one or more distal axial hole(s) **132** to allow fluid to be pushed out the distal portion of alternating-flow catheter **120.** Some embodiments combine one or more of the following: drainage port **156,** return port **158,** and hub with hemostatic valve **159.** In come embodiments, the check valves **190** and **192** are functionally situated within the connecting tubing or control system, instead of alternating-flow catheter **120.**

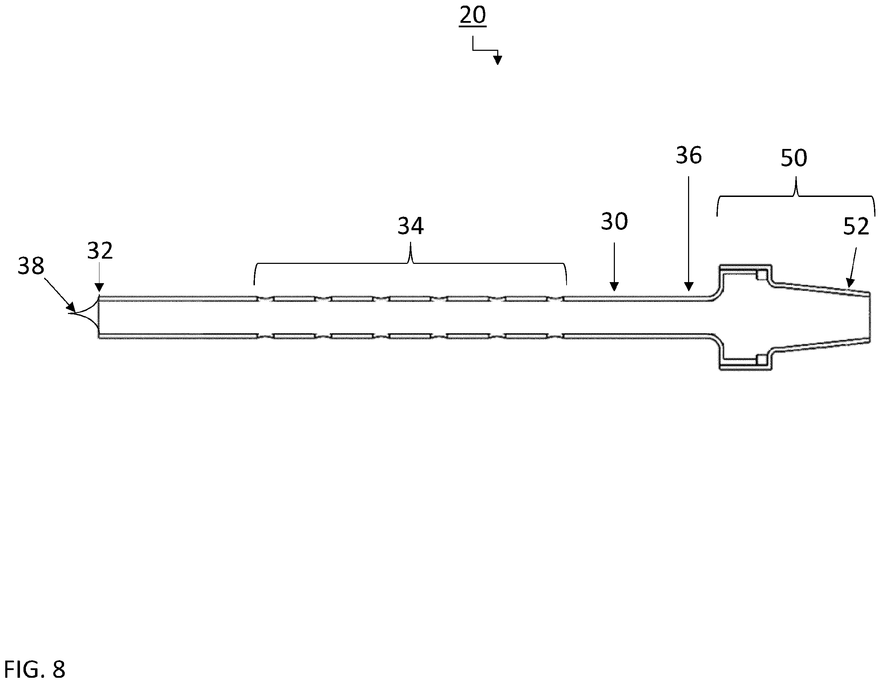

Fig. **8** generally illustrates another embodiment of part of the present disclosure. Under this embodiment, alternating-flow catheter **20** achieves near-maximal inner functional cross-sectional area usage for both drainage and return without an alternating-flow control mechanism that closes or opens the proximal holes **34** of outer catheter **30.** In embodiments rather, alternating-flow catheter **20** is designed (e.g., hold size, hole placement, hole shape, number of holes) such that its flow dynamics facilitate the preferential withdrawal of fluid from proximal hole(s) **34** during the drainage phase and the preferential ejection of fluid via distal axial hole(s) **32** during the return phase. Some embodiments do and some do not include check valve **38.** In some embodiments, alternating-flow catheter **20** does not have special flow dynamics, but recirculation is minimal enough (e.g., due to surrounding patient vascular anatomy) to allow sufficient device function.

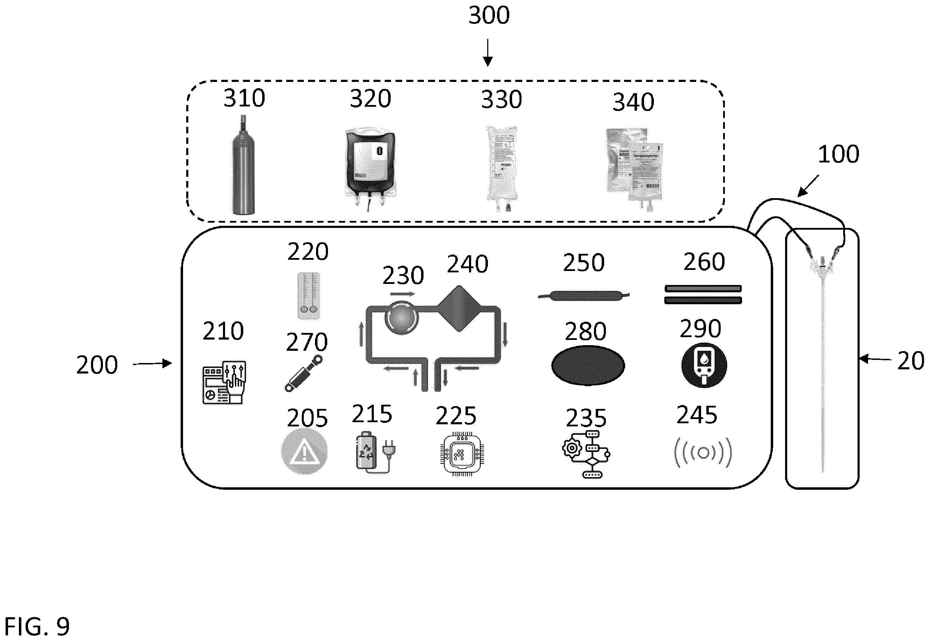

Fig. **9** generally illustrates another embodiment of part of the present disclosure. Under this embodiment, a system including an alternating-flow catheter **20,** connecting tubing 100, control system **200,** and one or more inputs and/or consumables **300** is shown. Control system **200** includes one or more of the following: user interface(s) **210,** gas blender(s) **220,** pump(s) **230,** oxygenator(s) **240,** heat-exchanger(s) **250,** perfusion circuit(s) and/or filter(s) **260,** actuator(s) **270,** reservoir(s) **280,** blood analyzer(s) **290,** alarm(s) **205,** battery(ies) **215,** processor(s) **225,** control algorithm(s) **235,** and/or sensor(s) **245.** Inputs and/or consumables **300** includes one or more of the following: oxygen **310,** blood product(s) **320,** fluids **330,** and/or medications **340.**

In embodiments, control system **200** is one of the many traditional ECLS systems connected via tubing to an intravascular catheter. Many of the traditional ECLS systems are essentially a single loop of blood being drained and then returned to the body utilizing a single pump and single reservoir. In embodiments, control system **200** is initially a standard ECLS system that is modified for use with alternating-flow catheter **20.** Traditional ECLS systems utilize continuous flow and thus to be used as disclosed herein must be modified to deliver alternating flow between oxygenated blood returned to the body and deoxygenated blood drained from the body through a single alternating-flow catheter **20.** In embodiments, this is achieved by one or more of the following: one or more additional reservoir(s) **280** (e.g., to allow for blood to build up in the system between alternating flows), one or more distal actuator(s) **270** (e.g., near alternating-flow catheter **20** that alternates flow between drainage and return to alternating-flow catheter **20**), a 3-way stopcock or related mechanism to alternate the fluid pathway from external tubing into the catheter, one or more additional pump(s) **230,** and/or one or more additional perfusion circuit(s) and/or filter(s) **260.**

In embodiments, blood is drained (i.e., withdrawn from the body) through alternating-flow catheter **20** and proceeds to control system **200** through connecting tubing **100.** The blood can be retained in control system **200** in a drainage reservoir **280.** In embodiments, one pump **230** causes blood to be pulled into the system and then pumped through oxygenator(s) **240** (e.g., to add oxygen and/or remove carbon dioxide from the blood). This can utilize the use of inputs and/or consumables **300** (e.g., oxygen **310,** heparin, fluids). In embodiments, this occurs in conjunction with gas blender(s) **220,** which adjusts the levels of oxygen and/or carbon dioxide in the system. Blood can then be retained in a return reservoir **280.** In embodiments, there is a second pump **230** that causes blood to be pushed pack through alternating-flow catheter **20.** In embodiments, heatexchanger(s) **250** warms the blood before placement back into the body. In embodiments, perfusion circuit(s) and/or filter(s) **260** filters the blood and/or returns it to the return connecting tubing 100. In embodiments, the user interacts with control system **200** using user interface(s) **210,** alarm(s) **205** can be set to notify the user of specific parameters, actuator(s) **270** functions to cause the device to operate, battery(ies) **215** and/or a wall plug function to provide electricity to the system, sensor(s) **245** function to sense system functionalities (e.g., blood pressure, blood oxygen level, blood carbon dioxide level, blood clotting), blood analyzer(s) **290** tests the blood for one or more characteristics (e.g., hemoglobin, glucose, lactate, pH), and processor(s) **225** utilizes control algorithm(s) **235** to control the system.

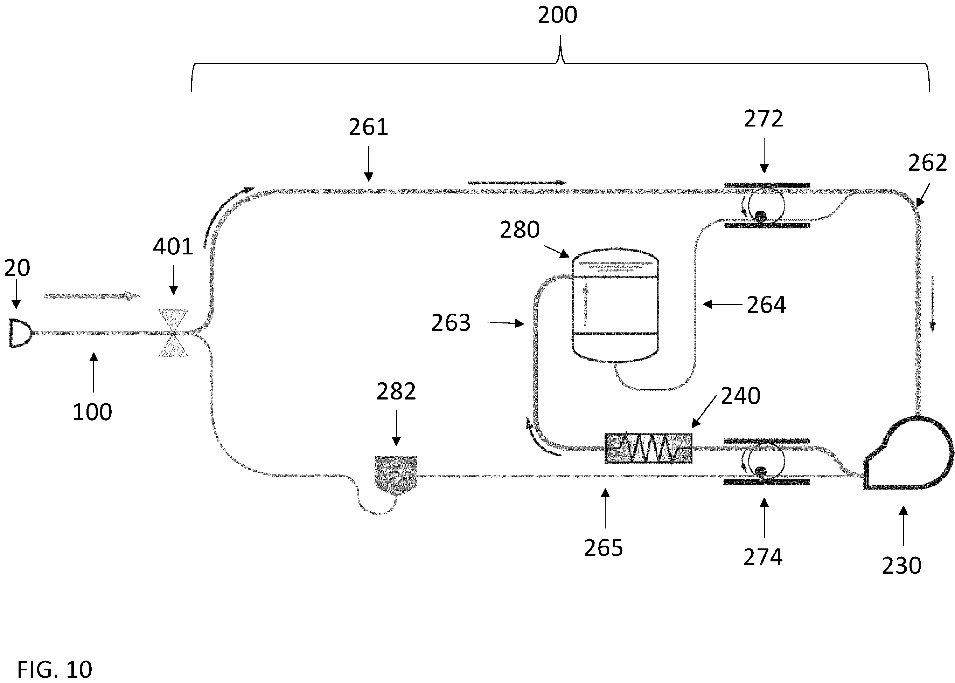

Figs. **10-11** generally illustrate another embodiment of part of the present disclosure. Fig. **10** shows a catheter-related medical device with alternating-flow catheter **20,** connecting tubing **100,** and control system **200** in the withdrawal phase draining blood from the body. In this embodiment, blood is drained (i.e., withdrawn from the body) through alternating-flow catheter **20** and proceeds to control system **200** through connecting tubing **100.** Connecting tubing **100** connects to control system **200** via 3-way stopcock or related mechanism **401.** Blood then travels through a drainage circuit loop consisting of tubing sections **261, 262, 263,** and **264.** In this embodiment, blood is preferentially caused to travel through the drainage perfusion circuit loop by the settings of actuators **272** and **274** (e.g., which in some embodiments are pinch valves), which cause the relative resistance between tubing sections **261** and **264** and tubing sections **263** and **265** to favor flow through **261** and **263** (i.e., the drainage loop). Flow is powered by pump **230,** which causes blood to flow through actuator **272,** into pump **230,** through actuator **274** and oxygenator **240** into reservoir **280.** Blood then preferentially builds up in reservoir **280** during the drainage phase. Once a sensor on reservoir **280** determines that the volume in it has reached a desired level and/or amount based on a control algorithm, control system **200** switches from drainage to return phase.

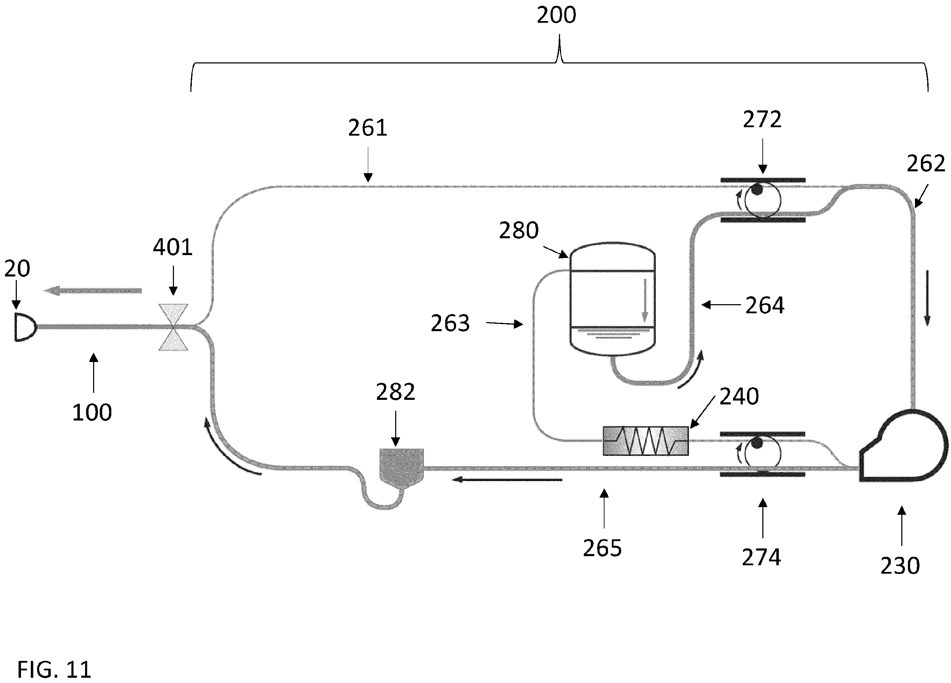

Fig. **11** shows the system in the return phase infusing blood and/or other medical fluids into the body. In this embodiment, travel through this perfusion circuit loop is powered by pump **230,** which causes blood to flow from reservoir **280** through actuator **272** into pump **230,** through pump **230,** actuator **274,** and bubble trap **282** into connecting tubing **100** and alternating-flow catheter **20** into the patient's body (e.g., femoral vein with catheter extension into the inferior portion of the IVC). In this embodiment, blood is preferentially caused to travel through the return perfusion circuit loop by the settings of actuators **272** and **274,** which cause the relative resistance between tubing sections **261** and **264** and tubing sections **263** and **265** to favor flow through **264** and **265** (i.e., the return loop). Blood thus preferentially is removed from reservoir **280** during this phase. Once a sensor on reservoir **280** determines that the volume in it has reached the desired level based on a control algorithm, control system **200** switches from return phase back to drainage phase. Thus, by alternating between the drainage and return phases, the system is able to provide alternating flow through the alternating-flow catheter **20.**

In embodiments, the 3-way stopcock or related mechanism **401** allows for priming of the system with blood and/or medical fluids (e.g., normal saline) during a setup phase (not shown). In embodiments, active actuation of the 3-way stopcock or related mechanism **401** is a distal actuator that additionally facilitates transitions between drainage and return phases. In embodiments, actuator **272** and actuator **274** are made up by more than one valve each (e.g., a pinch valve on tubing sections **261** and **264**), while in other embodiments they are a single pinch valve that alternates between pinching the tubing sections. In embodiments, the relative closure and/or resistance actuators **272** and **274** can be controlled from fully open, through gradations of partial closure, and/or to full closure of a loop. In embodiments, the control algorithm and/or user can apply different settings to the different loops for the drainage and return phases. In embodiments, the drainage and return phases actively and/or passively match with the alternating-flow catheter **20** such that drainage in the catheter preferentially occurs from the catheter's proximal portion and return through its distal portion.

In an embodiment, a catheter-based medical device can include at least one alternating-flow catheter configured to enable flow of fluid in one direction followed by flow of fluid in an opposite direction at least partially within a lumen of an outer catheter containing at least one distal axial hole and at least one proximal hole. The system can further include at least one control system having tubing configured to connect the at least one control system to the at least one alternating-flow catheter, at least one pump configured to drive a fluid through the at least one alternating-flow catheter, at least one reservoir configured to temporarily store a fluid and at least one sensor configured to determine an amount of the fluid in the reservoir.

In some embodiments, the at least one control system further comprises one or more oxygenators.

In some embodiments, the at least one control system is configured to execute one or more software algorithms to control flow of fluid through the alternating-flow catheter.

In some embodiments, the at least one control system further comprises one or more loop actuators configured to cause a change in direction of fluid flow in the at least one alternating-flow catheter.

In some embodiments, the at least one control system comprises at least two fluid circuit loops.

In some embodiments, the at least two fluid circuit loops include a drainage loop in which fluid is withdrawn from a body of a patient and stored in the reservoir and a return loop in which fluid flows from the reservoir and is infused into the body of the patient.

In some embodiments, the flow of fluid through the alternating-flow catheter includes a drainage phase in which fluid is withdrawn from a body of a patient and a return phase in which fluid is returned to the body of the patient.

In some embodiments, the at least one control system is configured to utilize the alternating-flow catheter for Extracorporeal Life Support.

In some embodiments, the at least one control system is configured to utilize the alternating-flow catheter for dialysis.

In some embodiments, the at least one alternating-flow catheter further comprises an inner catheter disposed within the outer catheter and including at least one distal axial hole and at least one proximal hole.

In some embodiments, in a withdrawal position the at least one proximal hole of the inner catheter is aligned with the at least one proximal hole of the outer catheter to enable flow of fluid into the lumen through the proximal holes and in a return position the at least one proximal hole of the inner catheter is misaligned with the at least one proximal hole of the outer catheter to prevent fluid from within the lumen from flowing therethrough.

In some embodiments, when the inner catheter is in the return position pressure within the alternating-flow catheter causes fluid to flow out of the at least one distal axial hole of the inner catheter and the at least one distal axial hole of the outer catheter.

In some embodiments, when the inner catheter is in the withdrawal position fluid does not flow into the at least one distal axial hole of the inner catheter or the at least one distal axial hole of the outer catheter.

In some embodiments, the alternating-flow catheter further includes an alternating-flow control mechanism configured to cause the inner catheter to move between the withdrawal position and the return position.

In some embodiments, the at least one proximal hole of the inner catheter is configured to be aligned with the at least one proximal hole of the outer catheter, and wherein the alternating-flow catheter is provided with flow dynamics that cause fluid to be drawn through the proximal holes during a drainage phase and fluid to be ejected through the distal axial holes during a return phase.

Various embodiments of systems, devices, and methods have been described herein. These embodiments are given only by way of example and are not intended to limit the scope of the disclosure. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the disclosure.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

For purposes of interpreting the claims, it is expressly intended that the provisions of 35 U.S.C. § 112(f) are not to be invoked unless the specific terms "means for" or "step for" are recited in a claim.

## Claims

1. A catheter-based medical device, comprising:
at least one alternating-flow catheter configured to enable flow of fluid in one direction followed by flow of fluid in an opposite direction at least partially within a lumen of an outer catheter containing at least one distal axial hole and at least one proximal hole; and
at least one control system including:
tubing configured to connect the at least one control system to the at least one alternating-flow catheter;
at least one pump configured to drive a fluid through the at least one alternating-flow catheter;
at least one reservoir configured to temporarily store a fluid; and
at least one sensor configured to determine an amount of the fluid in the reservoir.

2. The catheter-based medical device of claim 1, wherein the at least one control system further comprises one or more oxygenators.

3. The catheter-based medical device of claim 1 or claim 2, wherein the at least one control system is configured to execute one or more software algorithms to control flow of fluid through the alternating-flow catheter.

4. The catheter-based medical device of any of claims 1-3, wherein the at least one control system further comprises one or more loop actuators configured to cause a change in direction of fluid flow in the at least one alternating-flow catheter.

5. The catheter-based medical device of any of claims 1-4, wherein the at least one control system comprises at least two fluid circuit loops.

6. The catheter-based medical device of claim 5, wherein the at least two fluid circuit loops include a drainage loop in which fluid is withdrawn from a body of a patient and stored in the reservoir and a return loop in which fluid flows from the reservoir and is infused into the body of the patient.

7. The catheter-based medical device of any claims 1-6, where the flow of fluid through the alternating-flow catheter includes a drainage phase in which fluid is withdrawn from a body of a patient and a return phase in which fluid is returned to the body of the patient.

8. The catheter-based medical device of any of claims 1-7, wherein the at least one control system is configured to utilize the alternating-flow catheter for Extracorporeal Life Support or dialysis.

9. The catheter-based medical device of any of claims 1-8, wherein the at least one alternating-flow catheter further comprises an inner catheter disposed within the outer catheter and including at least one distal axial hole and at least one proximal hole.

10. The catheter-based medical device of claim 9, wherein in a withdrawal position the at least one proximal hole of the inner catheter is aligned with the at least one proximal hole of the outer catheter to enable flow of fluid into the lumen through the proximal holes and in a return position the at least one proximal hole of the inner catheter is misaligned with the at least one proximal hole of the outer catheter to prevent fluid from within the lumen from flowing therethrough.

11. The catheter-based medical device of claim 10, wherein when the inner catheter is in the return position pressure within the alternating-flow catheter causes fluid to flow out of the at least one distal axial hole of the inner catheter and the at least one distal axial hole of the outer catheter.

12. The catheter-based medical device of claim 10, wherein when the inner catheter is in the withdrawal position fluid does not flow into the at least one distal axial hole of the inner catheter or the at least one distal axial hole of the outer catheter.

13. The catheter-based medical device of claim 11, wherein the alternating-flow catheter further includes an alternating-flow control mechanism configured to cause the inner catheter to move between the withdrawal position and the return position.

14. The catheter-based medical device of claim 9, wherein the at least one proximal hole of the inner catheter is configured to be aligned with the at least one proximal hole of the outer catheter, and wherein the alternating-flow catheter is provided with flow dynamics that cause fluid to be drawn through the proximal holes during a drainage phase and fluid to be ejected through the distal axial holes during a return phase.

15. An alternating-flow catheter for use with the catheter-based medical device of any of claims 1-14.
